# EUROPÄISCHE PATENTSCHRIFT

(11) **EP 2 055 260 B1**
(45) Veröffentlichungstag und Bekanntmachung des Hinweises auf die Patenterteilung: **01.06.2016**
(21) Anmeldenummer: 08166141.5
(22) Anmeldetag: 08.10.2008
(51) Int. Cl.: A61C 13/20

(54) **Muffel**
Sleeve
Moufle

(30) Priorität: 05.11.2007 AT 17332007
(43) Veröffentlichungstag der Anmeldung: 06.05.2009
(73) Patentinhaber: Ivoclar Vivadent AG, 9494 Schaan (LI)
(72) Erfinder: Gritsch, Harald, 6182 Gries, (AT); Wörishofer, Max, 6410 Telfs, (AT); Zobler, Christoph,, 6071 Aldrans, (AT); Stampfer, Thomas,, 6806 Feldkirch-Tosters, (AT); Foser, Hans-Peter, 9496 Balzers (LI)
(74) Vertreter: Splanemann

(56) Entgegenhaltungen:
- EP-A- 1 093 769
- EP-A- 1 484 031

## Beschreibung

Die Erfindung betrifft eine Muffel gemäß dem Oberbegriff des Anspruch 1 und ein Verfahren gemäß dem Oberbegriff des Anspruchs 12.

Muffeln sind Einsätze für Öfen und schützen ein zu brennendes Objekt, wie zum Beispiel ein dentales Restaurationsteil vor Flammen oder einer direkten Heizelementstrahlung, die vom Ofen ausgeht.

Eine Muffel weist gewöhnlich einen Vorpressraum und mindestens einen Formhohlraum auf, der mit dem Vorpressraum über mindestens einen Verbindungskanal verbunden ist.

Der Vorpressraum ist zur Aufnahme eines zu verpressenden Materials vorgesehen. Dieses wird im Vorpressraum solange erhitzt bis es zu fließen beginnt, und danach in den Verbindungskanal gedrückt. Das Material wird gewöhnlich in Form von Rohlingen bereitgestellt.

Der Formhohlraum besitzt die Form des zu brennenden Objektes und nimmt das fließende Rohlingsmaterial unter hohem Druck auf. Wenn der Formhohlraum vollständig mit Rohlingsmaterial ausgefüllt ist, wird die Muffel und somit das Rohlingsmaterial im Formhohlraum gekühlt, und die Muffel wird entformt, um das fertige Produkt zu entnehmen.

In der Praxis zeigen sich jedoch am gebrannten Objekt immer wieder Risse, die insbesondere bei dentalen Restaurationsteilen äußerst problematisch sind, da diese Risse Hohlräume bilden. Darin sammeln sich Keime und andere Verunreinigungen an, die das dentale Restaurationsteil mit der Zeit beschädigen oder sogar zerstören. Aber auch ein allgemeines gebranntes Objekt wird durch derartige Risse in seiner Festigkeit und damit in seiner beeinträchtigt.

Ein weiteres Problem bei den eingangs beschriebenen Muffeln ist eine Totzeit zwischen Aufheizvorgang und dem eigentlichen Fließvorrang. Der Aufheizvorgang ist abgeschlossen, sobald das Rohlingsmaterial schmilzt. Der eigentliche Fließvorgang beginnt, sobald das geschmolzene Rohlingsmaterial in den Verbindungskanal eindringt. In der Praxis zeigt sich jedoch, dass sich der eigentliche Fließvorgang nicht unmittelbar an den Aufheizvorgang anschließt. Die Erfahrung zeigt, dass diese Totzeit zwischen dem Aufheizvorgang und dem eigentlichen Fließvorgang die Qualität des zu brennenden Objektes reduziert. Weiterhin muss während dieser Totzeit Energie aufgebracht werden, die das Rohlingsmaterial im geschmolzenen Zustand hält. Diese aufgebrachte Energie trägt jedoch nicht zur Herstellung des zu brennenden Objekts bei und reduziert daher den Wirkungsgrad des gesamten Systems.

Letztlich muss die Muffel beim Entformen und beim Entnehmen des gebrannten Objekts leicht gedreht werden, damit das Material des gebrannten Objekts nicht bricht. Diese technische Bedingung reduziert die Gestaltungsfreiheit der Rohlinge und des Vorpressraumes, da ein Drehen der Muffel nur dann möglich ist, wenn Rohling und Presskanal eine bestimmte Form aufweisen.

Es ist bekannt geworden in einer Muffel einen Pressraum mit einem kleeblattförmigen Querschnitt auszubilden, wie z.B. in EP 1 484 031 A1 dargestellt, in dem eine Vielzahl von Keramikpelletts aufnehmbar sind, die dann zu einem Zahnrestaurationsteil verpresst werden. Da Pelletts Schüttgüter sind und deshalb in den Pressraum eingeschüttet werden, ist ihre Lage zueinander unbestimmt. Dies erhöht die Anzahl der Zwischenräume im Presskanal und folglich die oben beschriebene Totzeit zwischen Aufheizvorgang und Fließvorgang.

Demgegenüber liegt der Erfindung die Aufgabe zugrunde, eine Muffel gemäß dem Oberbegriff des Anspruchs 1 und ein verfahren anordnung gemäß dem Oberbegriff des Anspruchs 12 zu schaffen, mit denen beim Pressen und beim Brennen eines Produktes weniger Risse entstehen.

Erfindungsgemäß ist es vorgesehen, einen Vorpressraum derart auszugestalten, dass wenigstens zwei Rohlinge nebeneinander im Vorpressraum aufnehmbar sind. Durch diese Maßnahme wird das Verhältnis zwischen einer Querschnittsfläche des Vorpressraumes und einer Querschnittsfläche des Verbindungskanals zum Formhohlraum drastisch erhöht. Mit anderen Worten erfolgt der Übergang zwischen Vorpressraum und Verbindungskanal noch aprupter, wodurch der Druck auf das geschmolzene Rohlingsmaterial beim Drücken in den Verbindungskanal sehr stark erhöht wird. Überraschenderweise zeigt sich, dass das geschmolzene Material im Verbindungskanal durch diesen hohen Druck eine stärkere Verwirbelung erfährt und dadurch besser durchmischt wird als mit herkömmlichen Verfahren. Im Ergebnis ist das geschmolzene Rohlingsmaterial heterogener und gleichmäßiger in seiner Struktur. Dies zeigt sich insbesondere am gebrannten Objekt, das nach der Herstellung weniger Spannungsrisse aufweist, als Objekte die mit herkömmlichen Muffeln gebrannt werden.

Die Durchmischung des geschmolzenen Rohlingsmaterials kann verbessert werden, wenn der Verbindungskanal vom Zulauf an der Seite zum Vorpressraüm zu einem Ablauf an der Seite zum Formhohlraum mindestens teilweise zunimmt. Dadurch kann der Druck auf das Rohlingsmaterial beim Durchlaufen des Verbindungskanals weiter erhöht werden, so dass dieses beim Eintritt in den Formhohlraum, wenn der Druck schlagartig nachläßt, noch stärker verwirbelt und damit noch besser durchmischt wird.

Weiter kann im Verbindungskanal eine Kammer vorgesehen sein, durch die ebenfalls ein schlagartiges Nachlassen des Drucks und damit eine Steigerung der Verwirbelung erreicht wird. Somit wird durch die Verwendung einer kleinen Kammer im Verbindungskanal das geschmolzene Rohlingsmaterial zweimal entspannt, verwirbelt und damit stärker durchmischt.

Um das Eintreten in den Verbindungskanal zu erleichtern, kann der Verbindungskanal am Zulauf eine Phase aufweisen, die trichterförmig das geschmolzene Rohlingsmaterial aufnimmt.

Um die Verwirbelung weiter zu steigern, kann das Oberflächenprofil des Verbindungskanals unregelmäßig sein. Dies kann dadurch erreicht werden, dass das Oberflächenprofil besonders aufgeraut und/oder wellig ausgebildet ist. Durch die aufgeraute Oberfläche, also die aufgeraute Wand im Verbindungskanal reibt die geschmolzene Rohlingsmasse ständig an dieser und verwirbelt so weiter. Durch das wellige Oberflächenprofil werden in gleichmäβigen Abständen Kammern ausgebildet, die in regelmäßigen Abständen Druck auf das Rohlingsmaterial aufbauen, der danach schlagartig wieder nachlässt. Somit kann der oben beschriebene Grundgedanke der Erfindung im Verbindungskanal mehrere Male wiederholt werden, wodurch die Durchmischung weiter verbessert wird. Der Vorpressraum kann weiter derartig ausgestaltet sein, dass ein Stapeln von mehreren Rohlingen möglich ist. Dadurch kann mehr Material zum Verpressen bereitgestellt werden, so dass auch größere zu brennende Produkte wie ein dentaler Kieferquadrant mit der Muffel herstellbar sind.

Der Querschnitt des Vorpressraumes kann rund, oval, elliptisch oder mehreckig sein, und so an die Form der im Vorpressraum ausgenommenen Rohlinge angepasst werden. Dadurch werden, Lücken zwischen einer Wand des Vorpressraumes und den darin aufgenommenen Rohlingen oder zwischen den Rohlingen selbst verkleinert, wodurch das Ausfüllen dieser Lücken it geschmolzenen Rohlingsmaterial vor dem eigentlichen Fließvorgang ebenfalls verkürzt wird. Diese Zeit zum Füllen der Lücken kann weiter verkürzt werden, wenn die Rohlinge an eine Form des Vorpressraumes so gut wie möglich angepasst werden. Beispielsweise können bei einem mehreckigen Vorpressraum mehreckige Rohlinge verwendet werden. Um eine optimale Stabilität der Muffel zu gewährleisten kann die Wandstärke der Muffel zwischen der Hälfte und dem 1,5-fachen des Durchmessers des Vorpressraumes liegen. Beste Ergebnisse haben sich gezeigt, wenn die Wandstärke ca. genauso groß wie der Durchmesser des Vorpressraumes ist.

In einer weiteren vorteilhaften Ausgestaltung ist es vorgesehen, dass in dem Presskanal drei Rohlinge aufnehmbar sind.

In einer weiteren vorteilhaften Ausgestaltung ist es vorgesehen, dass die Rohlinge linienförmig aneinander liegen.

In einer weiteren vorteilhaften Ausgestaltung ist es vorgesehen, dass der Querschnitt des Presskanals rund oder im Wesentlichen oval, elliptisch oder mehreckig ist.

In einer weiteren vorteilhaften Ausgestaltung ist es vorgesehen, dass die Rohlinge den Presskanal gemeinsam, im Wesentlichen vollständig, ausfüllen.

In einer weiteren vorteilhaften Ausgestaltung ist es vorgesehen, dass eine von im Presskanal nebeneinander liegenden Rohlingen gebildete Rohling-Außenform an die Querschnittsform des Presskanals angepasst ist.

In einer weiteren vorteilhaften Ausgestaltung ist es vorgesehen, dass in dem Presskanal zwei Ebenen vor jeweils mindestens zwei Rohlingen aufnehmbar sind.

In einer weiteren vorteilhaften Ausgestaltung ist es vorgesehen, dass in dem Presskanal zwei Ebenen von jeweils drei Rohlingen aufnehmbar sind.

In einer weiteren vorteilhaften Ausgestaltung ist es vorgesehen, dass in dem Presskanal zwei Ebenen mit einer unterschiedlichen Anzahl von unterschiedlich großen Rohlingen aufnehmbar sind.

In einer weiteren vorteilhaften Ausgestaltung ist es vorgesehen, dass vor dem Pressvorgang wenigstens zwei Rohlinge nebeneinander und aneinander liegend in einen Presskanal eingesetzt werden.

In einer weiteren vorteilhaften Ausgestaltung ist es vorgesehen, dass vor dem Pressvorgang drei Rohlinge nebeneinander und aneinander liegend in den Presskanal eingesetzt werden.

Weitere Vorteile, Einzelheiten, und Merkmale ergeben sich aus der nachfolgenden Beschreibung mehrerer Ausführungsbeispiele anhand der Zeichnungen.

Es zeigen:
- Fig. 1: Ein Querschnitt einer erfindungsgemäßen Muffel mit drei aufgenommenen Rohlingen und einem Pressstempel;
- Fig. 2: Ein erstes bis drittes Ausführungsbeispiel für einen Verbindungskanal in der Muffel;
- Fig. 3: Ein viertes bis sechstes Ausführungsbeispiel für einen Verbindungskanal in der Muffel;
- Fig. 4: Einen Querschnitt der erfindungsgemäßen Muffel;
- Fig. 5: Ein Schrägbild eines ersten Ausführungsbeispiels der erfindungsgemäßen Muffel mit darin aufgenommenen Rohlingen;
- Fig. 6: Ein Schrägbild eines zweiten Ausführungsbeispiels der erfindungsgemäßen Muffel mit darin aufgenommenen Rohlingen, und
- Fig. 7: Ein Schrägbild eines dritten Ausführungsbeispiels der Erfindungsgemäßen Muffel mit darin aufgenommenen Rohlingen.

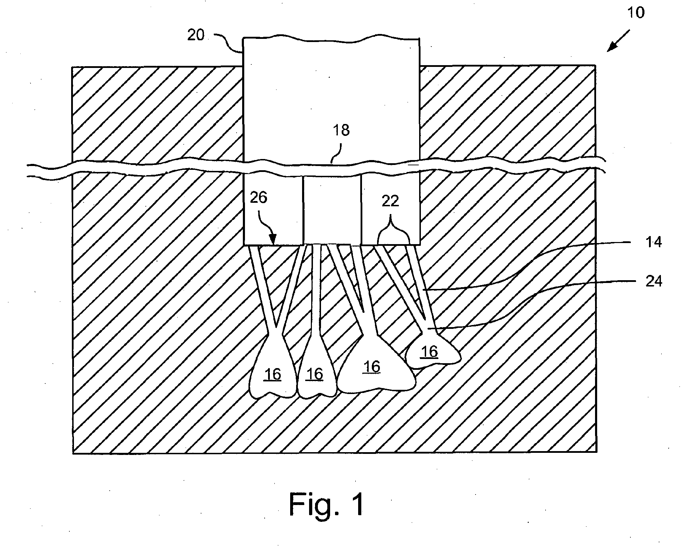

Gemäß Fig. 1 weist eine erfindungsgemäße Muffel 10 einen Vorpressraum 12, mehrere Verbindungskanäle 14 und mehrere Formhohlräume 16 auf. Im Vorpressraum 12 der Muffel 10 sind drei Rohlinge 18 nebeneinander angeordnet, und werden von einem Pressstempel 20 abgedeckt. Die Rohlinge 18 liegen auf einem Zugang 22 zum Verbindungskanal 14 auf.

Im Einsatz werden die Rohlinge 18 erwärmt und schmelzen so. Die geschmolzene Rohlingsmasse dringt über den Zugang 22 in den Verbindungskanal 14 ein, wobei die geschmolzene Rohlingsmasse dann über einen Austritt 24 aus dem Verbindungskanal 14 austritt und in den Formhohlraum 16 fließt.

Wie aus der Fig. 1 unmittelbar ersichtlich, wird durch die mehreren nebeneinander gelagerten Rohlinge 18 eine große Bodenfläche 26 der Muffel 10 abgedeckt. Demgegenüber steht jedoch nur eine sehr kleine Fläche für den Zugang 22 zum Verbindungskanal 14. So wird mit dem Pressstempel 20 im Vorpressraum 12 ein sehr hoher Druck aufgebaut. Beim Austritt der Rohlingmasse aus dem Verbindungskanal 14 lässt dieser Druck schlagartig nach, wodurch die Rohlingsmasse gut verwirbelt und das geschmolzene Material der Rohlinge besser durchmischt wird.

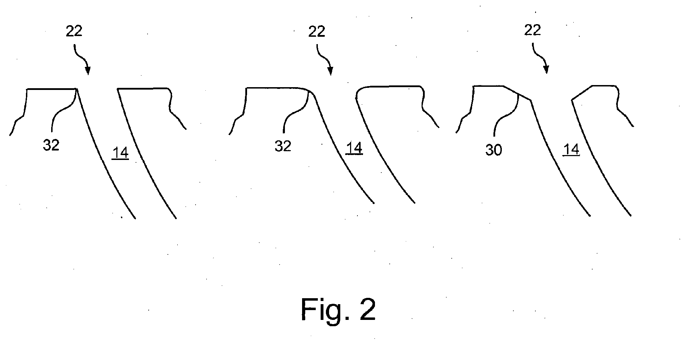

Die Durchmischung des geschmolzenen Materials der Rohlinge 18 kann durch die Ausgestaltung der Verbindungskanäle 14 weiter verbessert werden. Wie in Fig. 2 gezeigt, können die Kanten 32 am Zutritt 22 des Verbindungskanals 14 eine Abrundung 28 oder eine Phase 30 aufweisen.

Dadurch wirkt der Zutritt 22 des Verbindungskanals 14 wie ein Trichter, wodurch die Rohlingsmasse im Verbindungskanal 14 weiter verdickt und der Druck im Verbindungskanal weiter erhöht wird, so dass die Durchmischung des geschmolzenen Rohlingsmaterials beim Austritt aus dem Verbindungskanal 14 weiter verbessert wird.

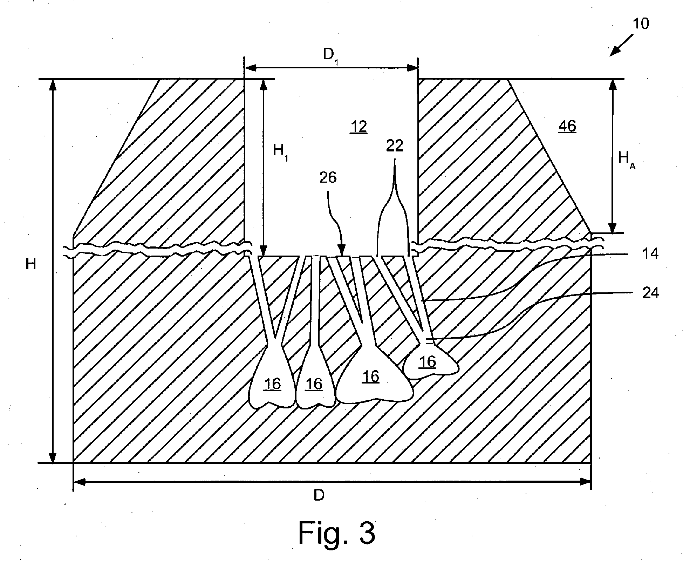

Gemäß Fig. 3 kann aber auch der Verbindungskanal selbst in seiner Form zur besseren Durchmischung des geschmolzenen Materials der Rohlinge 18 beitragen.

In einer ersten Ausführung kann beispielsweise die Oberfläche 34 des Verbindungskanals 14 aufgeraut sein. Im Extremfall wird dies durch eine wellige Oberflächenform erreicht. Somit verjüngt und verbreitet sich der Querschnitt des Verbinungskanals regelmäßig, wodurch ständig Druck auf das geschmolzene Rohlingsmaterial aufgebaut wird, der dann wieder schlagartig nachlässt. Dadurch wird das geschmolzene Material der Rohlinge 18 ständig verwirbelt und so besser durchmischt.

Alternativ kann im Verbindungskanal 14 eine kleine Kammer 36 eingebaut sein, mit der ebenfalls ein regelmäßiger Druck ab- und wiederaufbau erreicht wird, so dass das Material durch die Kammer 36 einer regelmäßigen Verwirbelung ausgesetzt wird, die das geschmolzene Material der Rohlinge 18 weiter durchmischt.

In einer weiteren alternativen Ausführung des Verbindungskanals 14 kann sich ein Querschnitt 38 des Verbindungskanals 14 nach dem Zutritt 22 ständig verbreitern.

In dieser Ausführung fällt der Druck auf das geschmolzene Rohlingsmaterial schon im Verbindungskanal ab, so dass die Verwirbelung und Durchmischung delbst dann noch gegeben ist, wenn der Formhohlraum schon fast vollständig mit geschmolzenen Rohlingsmaterial ausgefüllt ist.

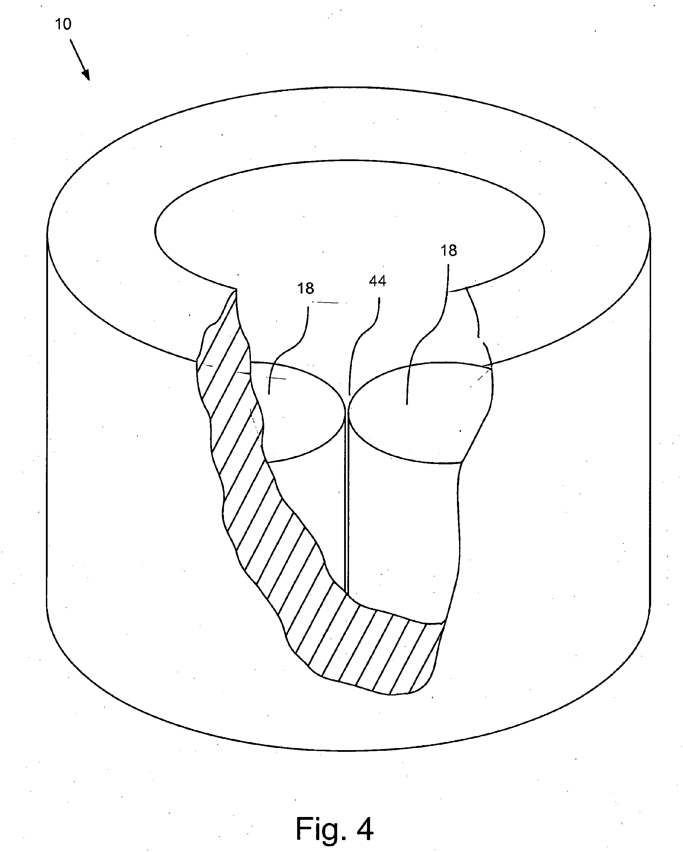

In Fig. 4 sind einige Abmessungen der Muffel 10 nicht maßstabsgetreu skitziert. Die Muffel 10 weist einen Bodenbereich 40 und eine Wand 42 auf. Die Stärke des Bodenbereiches ist so gewählt, dass sie ungefähr 0,5 bis 1,5 mal so stark ist wie eine Tiefe h1 des Vorpressraumes. Die Wand 42 der Muffel ist ebenfalls so ausgestaltet, dass sie 0,5 bis 1,5 mal so stark ist wie eine Breite des Vorpressraumes 12. Vorzugsweise ist der Bodenbereich genauso stark wie der Vorpressraum 12 tief ist. Ebenso kann die Wand 42 genauso stark wie der Vorpressraum 12 breit sein.

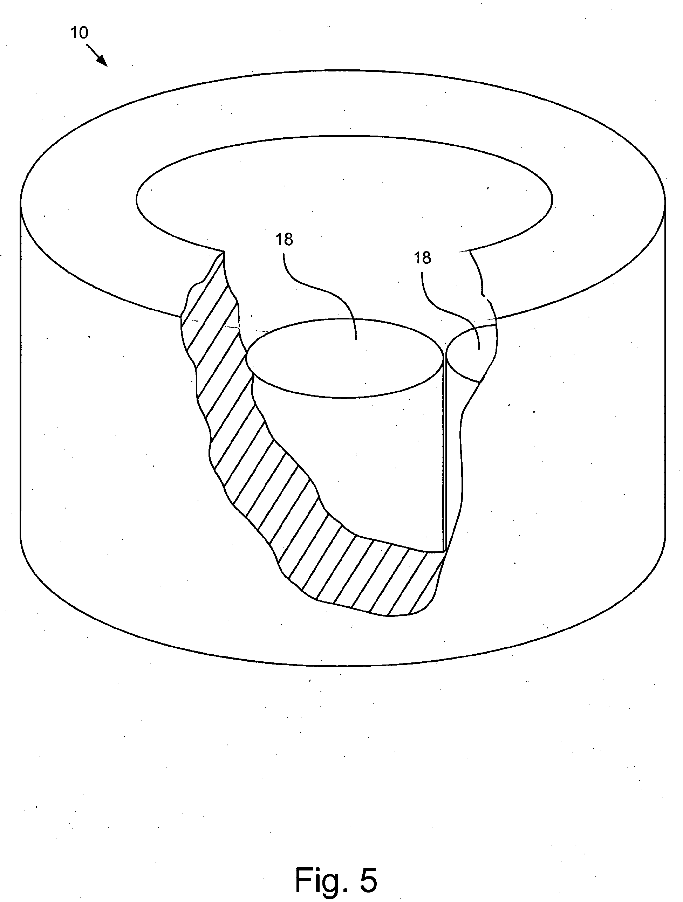

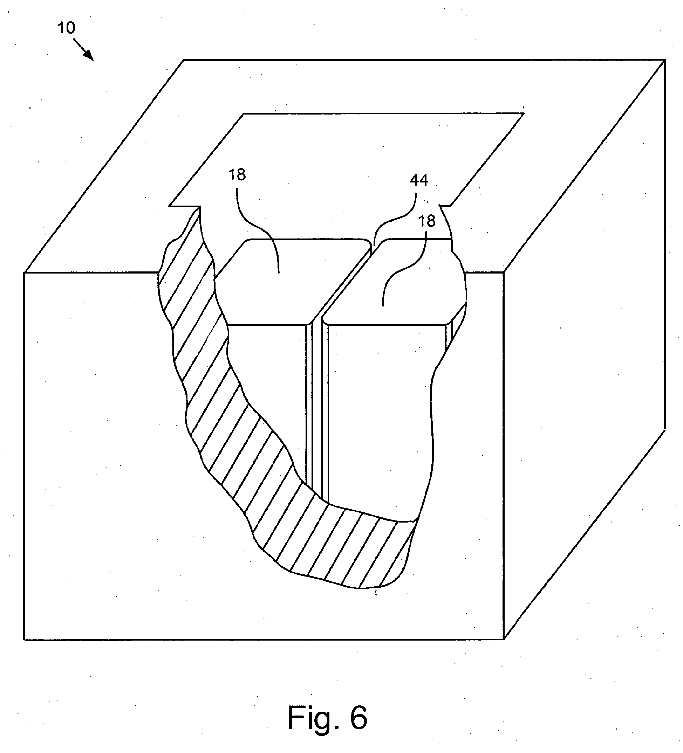

In den Fig. 5 bis Fig. 7 sind Ausführungsbeispiele für die Muffel 10 mit darin aufgenommenen Rohlingen 18 gezeigt. Danach kann die Muffel 10 oval aufgebaut sein, so dass zwei Rohlinge nebeneinander aufnehmbar sind. Dies hat jedoch zum Nachteil dass ein recht großer Zwischenraum 44 entsteht, so dass beim Erwärmen der Muffel 10 eine recht lange Zeit verstreicht, bis alle Zwischenräume 44 mit geschmolzenem Material der Rohlinge 18 gefüllt sind und der eigentliche Pressvorgang startet. Um diesem Problem zu begegnen, können, wie in Fig. 6 gezeigt, Rohlinge 18 verschiedener Größen verwendet werden. Dadurch kann der Zwischenraum 44 verkleinert werden, wodurch die Zeit beim Erwärmen der Muffel bis zum Starten des eigentlichen Pressvorgangs verkürzt wird.

Besonders effektiv lassen sich die Zwischenräume 44 vermeiden, wenn die Rohlinge 18 gemäß Fig. 7 einen viereckigen Querschnitt aufweisen, und der Vorpressraum 12 ebenfalls viereckig aufgebaut ist. Dadurch lassen sich die Rohlinge 18 mit dem kleinstmöglichen Zwischenraum 44 aufbauen, so dass die Aufwärmzeit bis zum eigentlichen Start des Pressvorgangs mit der Muffel minimiert werden kann.

## Patentansprüche

1. Muffel (10), insbesondere zur Herstellung von keramischen Dentalrestaurationen, mit:
einem der Aufnahme eines Pressrohlings (18) dienenden Presskanal (12); und
mindestens einem Formhohlraum (16), der mit dem Presskanal (12) über mindestens einen Verbindungskanal (14) verbunden ist;
wobei der Presskanal (12) geeignet ist, mindestens zwei Rohlinge (18) aneinander anliegend aufzunehmen, und
der Presskanal derart geformt ist, dass die Rohlinge (18) nebeneinander liegend anordnebar sind; **dadurch gekennzeichnet, dass**
der Presskanal (12) im Wesentlichen vollständig von den Rohlingen (18) ausfüllbar ist, wobei geschmolzenes Rohlingsmaterial durch den mindestens einen Verbindungskanal (14) in den Formhohlraum (16) pressbar ist, wobei der mindestens eine Verbindungskanal (14) derart ausgestaltet ist, dass das geschmolzene Rohlingsmaterial im Verbindungskanal (14) durchmischbar ist.

2. Muffel (10) nach Anspruch 1, **dadurch gekennzeichnet, dass** der mindestens eine Verbindungskanal (14) einen trichterförmigen Zutritt (22) aufweist.

3. Muffel nach Anspruch 1, **dadurch gekennzeichnet, dass** der mindestens eine Verbindungskanal (14) eine aufgeraute Oberfläche (34) und insbesondere eine wellige Oberflächenform aufweist.

4. Muffel nach Anspruch 1, **dadurch gekennzeichnet, dass** in dem mindestens einen Verbindungskanal (14) eine kleine Kammer (36) vorgesehen ist, so dass eine regelmäßige Verwirbelung des geschmolzenen Rohlingsmaterials im Verbindungskanal (14) realisierbar ist.

5. Muffel (10) nach einem der vorhergehenden Ansprüche, **dadurch gekennzeichnet, dass** in dem Presskanal (12) drei Rohlinge (18) aufnehmbar sind.

6. Muffel (10) nach einem der vorhergehenden Ansprüche, **dadurch gekennzeichnet, dass** der Presskanal (12) derart dimensioniert ist, dass die Rohlinge (18) linienförmig aneinander liegen können.

7. Muffel (10) nach einem der vorstehenden Ansprüche, **dadurch gekennzeichnet, dass** der Querschnitt des Presskanals (12) rund, oval, elliptisch oder mehreckig ist oder die Form eines Langlochs oder eines Rechtecks aufweist.

8. Muffel (10) nach einem der vorstehenden Ansprüche, **dadurch gekennzeichnet, dass** eine von im Presskanal (12) nebeneinander liegenden Rohlingen (18) gebildete Rohling-Außenform an die Querschnittform des Presskanals (12) angepasst ist.

9. Muffel (10) nach einem der vorstehenden Ansprüche, **dadurch gekennzeichnet, dass** in dem Presskanal (12) zwei Ebenen von jeweils mindestens zwei Rohlingen (18) aufnehmbar sind.

10. Muffel (10) nach einem der vorstehenden Ansprüche, **dadurch gekennzeichnet, dass** in dem Presskanal (12) zwei Ebenen von jeweils drei Rohlingen (18) aufnehmbar sind.

11. Muffel (10) nach einem der vorstehenden Ansprüche, **dadurch gekennzeichnet, dass** in dem Presskanal (12) zwei Ebenen mit einer unterschiedlichen Anzahl von unterschiedlich großen Rohlingen (18) aufnehmbar sind.

12. Verfahren zur Herstellung von keramischen Dentalrestaurationsteilen mit einer Muffel (10) gemäß einem der Ansprüche 1 bis 11, wobei vor dem Pressvorgang wenigstens zwei Rohlinge (18) aneinander anliegend in einen Presskanal (12) der Muffel (10) eingesetzt werden,
**dadurch gekennzeichnet, dass**
die Rohlinge (18) nebeneinander liegend angeordnet sind;
die Rohlinge (18) den Presskanal (12) gemeinsam, im Wesentlichen vollständig ausfüllen; und
geschmolzenes Rohlingsmaterial durch mindestens einen Verbindungskanal (12) in einen Formhohlraum (16) gepresst wird.

13. Verfahren zur Herstellung von keramischen Dentalrestaurationsteilen mit einer Muffel (10) gemäß Anspruch 12, **dadurch gekennzeichnet, dass** vor dem Pressvorgang drei Rohlinge (18) nebeneinander und aneinander liegend in den Presskanal (12) eingesetzt werden.

## Claims

1. A muffle (10), in particular for producing ceramic dental restorations, with:
a pressing channel (12) serving to receive a press blank (18); and
at least one mold cavity (16) that is connected to the pressing channel (12) via at least one connection channel (14);
wherein the pressing channel (12) is suitable for receiving at least two blanks (18) which abut on one another, and
wherein the pressing channel is shaped such that the blanks (18) can be arranged such that they are adjacent to one another; **characterized in that**
the pressing channel (12) can be filled up substantially completely by the blanks (18), wherein molten blank material can be pressed into the mold cavity (16) through the at least one connection channel (14), wherein the at least one connection channel (14) is configured such that the molten blank material can be intermixed in the connection channel (14).

2. The muffle (10) as claimed in claim 1, **characterized in that** the at least one connection channel (14) comprises a funnel-shaped access (22).

3. The muffle as claimed in claim 1, **characterized in that** the at least one connection channel (14) comprises a roughened surface (34) and in particular a wavy surface form.

4. The muffle as claimed in claim 1, **characterized in that** in the at least one connection channel (14) a small chamber (36) is provided such that regular vortexing of the molten blank material can be realized in the connection channel (14).

5. The muffle (10) as claimed in one of the preceding claims, **characterized in that** the pressing channel (12) is capable of receiving three blanks (18).

6. The muffle (10) as claimed in one of the preceding claims, **characterized in that** the pressing channel (12) is dimensioned such that the blanks (18) can abut linearly on one another.

7. The muffle (10) as claimed in one of the preceding claims, **characterized in that** the crosssection of the pressing channel (12) is round, oval, elliptic or polygonal or comprises the shape of an oblong hole or a rectangle.

8. The muffle (10) as claimed in one of the preceding claims, **characterized in that** a blank outer contour formed by blanks (18) which are adjacent to one another in the pressing channel (12) matches the cross-section of the pressing channel (12).

9. The muffle (10) as claimed in one of the preceding claims, **characterized in that** the pressing channel (12) is capable of receiving two levels of at least two blanks (18) each.

10. The muffle (10) as claimed in one of the preceding claims, **characterized in that** the pressing channel (12) is capable of receiving two levels of three blanks (18) each.

11. The muffle (10) as claimed in one of the preceding claims, **characterized in that** the pressing channel (12) is capable of receiving two levels of blanks (18), said blanks being different in number and different in size.

12. A method for producing ceramic dental restoration components with the aid of a muffle (10) as claimed in one of the claims 1 to 11, wherein prior to pressing at least two blanks (18) are inserted into a pressing channel (12) of the muffle (10) abutting on one another,
**characterized in that**
the blanks (18) are arranged adjacent to one another;
the blanks (18) fill up the pressing channel (12) substantially completely together; and
molten blank material is pressed through at least one connection channel (12) into a mold cavity (16).

13. The method for producing ceramic dental restoration components with the aid of a muffle (10) as claimed in claim 12, **characterized in that** prior to pressing three blanks (18) are inserted into the pressing channel (12) adjacent to and abutting on one another.

## Revendications

1. Moufle (10), en particulier pour la fabrication de restaurations dentaires en céramique, avec:
un canal de pressage (12) servant a accueillir un lingotin de pressée (18) ; et
au moins une cavité de moule (16), associée au canal de pressage (12) par au moins un canal de connexion (14) ;
où le canal de la pressage (12) est adapté pour accueillir, au moins deux lingotins de pressée (18) en appui les uns sur les autres et
le canal de pressage est formé de telle manière telle que les lingotins de pressée (18) peuvent être positionnés les uns contre les autres, **caractérisé en ce que** le canal de pressage (12) peut être essentiellement complètement rempli par les lingotins (18), où le matériel fondu d'un lingotin de pressée peut être pressée dans la cavité de moule (16) d'au moins un canal de connexion (14), où tel au moins un canal de connexion (14) est conçu de telle manière, que le matériel fondu d'un lingotin de pressée peut être mélangé dans le canal (14) de connexion.

2. Moufle (10) selon la revendication 1, **caractérisée en ce que** tel au moins un canal de connexion (14) dispose d'une entrée en forme d'entonnoir (22).

3. Moufle selon la revendication 1, **caractérisé en ce que** tel au moins un canal de connexion (14) présente une surface rugueuse (34) et en particulier une forme superficielle ondulée.

4. Moufle selon la revendication 1, **caractérisé en ce qu'**une petite chambre (36) est prévu dans tel au moins un canal de connexion (14), afin qu'un tourbillonnement modéré régulier du matériel fondu d'un lingotin de pressée dans le canal de connexion (14) puisse être réalisé.

5. Moufle (10) selon l'une des revendications précédentes, **caractérisé en ce que** trois lingotins de pressée (18) peuvent être accueillis dans le canal de pressage (12).

6. Moufle (10) selon l'une des revendications précédentes, **caractérisé en ce que** le canal de pressage (12) est dimensionné afin de permettre la disposition des lingotins de pressée (18) les uns contre les autres en forme de ligne.

7. Moufle (10) selon l'une des revendications précédentes, **caractérisé en ce que** la section transversale du canal de pressage (12) est ronde, ovale, elliptique ou polygonale ou prend la forme d'un trou oblong ou d'un rectangle.

8. Moufle (10) selon l'une des revendications précédentes, **caractérisé en ce qu'**un contour extérieur formé par des lingotins de pressée (18) contigus dans le canal de pressage (12) est adapté à la forme transversale du canal de pressage (12).

9. Moufle (10) selon l'une des revendications précédentes, **caractérisé en ce que** deux niveaux à au moins deux lingotins de pressée (18) peuvent être accueillis dans le canal de pressage (12).

10. Moufle (10) selon l'une des revendications précédentes, **caractérisé en ce que** deux niveaux à trois lingotins de pressée (18) peuvent être accueillis dans le canal de pressage (12).

11. Moufle (10) selon l'une des revendications précédentes, **caractérisé en ce que** deux niveaux avec un nombre variable de lingotins de pressée (18) de taille différentes peuvent être accueillis dans le canal de pressage (12).

12. Procédure pour la fabrication de pièces de restauration dentaire en céramique avec un moufle (10) selon l'une des revendications 1 à 11, où avant la procédure de pressage au moins deux lingotins de pressée (18) sont insérés dans un canal de pressage (12) du moufle (10), en appui les uns sur les autres, **caractérisée en ce que**,
les lingotins de pressée (18) sont disposés couchés les uns contre les autres,
les lingotins de pressée (18) remplissent le canal de pressage (12), essentiellement complètement ; et
du matériel fondu des les lingotins de pressée est pressé par au moins un canal de connexion (12) dans une cavité de moule (16).

13. Procédure pour la fabrication de pièces de restauration dentaire en céramique avec un moufle (10) selon la revendication 12, **caractérisé en ce qu'**avant la procédure de pressé trois blancs (18) contigus et les uns contre le autres sont insérés dans le canal de pressage (12).
